# EUROPEAN PATENT SPECIFICATION

(11) **EP 4 288 764 B1**
(45) Date of publication and mention of the grant of the patent: **09.07.2025**
(21) Application number: 22750613.6
(22) Date of filing: 01.02.2022
(51) Int. Cl.: G01N 21/76, G01N 21/64, G01J 1/58, C09K 11/00, G01N 33/58

(54) **HIGH-SENSITIVITY CHEMILUMINESCENCE DETECTION SYSTEMS AND METHODS**
HOCHEMPFINDLICHE CHEMILUMINESZENZDETEKTIONSSYSTEME UND -VERFAHREN
SYSTÈMES ET MÉTHODES DE DÉTECTION DE CHIMIOLUMINESCENCE À HAUTE SENSIBILITÉ

(30) Priority: 03.02.2021 US 202163145396 P
(43) Date of publication of application: 13.12.2023
(73) Proprietor: Siemens Healthcare Diagnostics, Inc., Tarrytown, NY 10591 (US)
(72) Inventor: MACH, Tivadar, 10179 Berlin (DE)
(74) Representative: Schweitzer, Klaus
(86) International application number: PCT/US2022/070461
(87) International publication number: WO 2022/170313

(56) References cited:
- EP-A1- 2 952 861
- WO-A1-2020/104769
- US-A1- 2001 035 947
- US-A1- 2002 055 181
- US-A1- 2012 264 107
- US-A1- 2015 118 690
- US-A1- 2017 038 514
- US-A1- 2017 346 556
- US-A1- 2018 275 056
- US-A1- 2020 008 716
- US-B1- 7 226 752
- US-B2- 9 255 885

## Description

### CROSS REFERENCE TO RELATED APPLICATION

This application claims the benefit of U.S. Provisional Patent Application No. 63/145,396, entitled "HIGH-SENSITIVITY CHEMILUMINESCENCE DETECTION SYSTEMS AND METHODS" filed February 03, 2021.

### FIELD

This disclosure relates to photodetection in immunoassays and more particularly, to chemiluminescent detections systems and methods configured to carry out measurements of luminescent light emissions that emanate from a reaction vessel.

### BACKGROUND

Chemiluminescence (CL) is defined as the emission of electromagnetic radiation caused by a chemical reaction to produce light. A chemiluminescence immunoassay (CLIA) is an assay that combines a chemiluminescence technique with an immunochemical reaction. For example, ATELLICA^{®} IM 1300, IMMULITE^{®} 1000, and ADVIA^{®} Centaur, available from the present assignee are instruments based on chemiluminescence immunoassays.

Similar to other labeled immunoassays (e.g., RIA, FIA, ELISA), CLIA systems utilize chemical probes that can generate luminescent light emission through a chemical reaction to quantify the analyte (component of interest). In some automated immunoassay systems, a sample container (e.g., a cuvette) containing extracted components (e.g., DNA probes) extracted from a specimen (e.g., a biological specimen such as blood serum or plasma or urine) that have been labeled to form labeled components that can be positioned at a desired location with the system. Thereafter, by causing a reaction, luminescent intensity readings can be obtained of the luminescent light emissions that emanate from the labeled components.

For example, in some embodiments, acridinium ester labels are used to label components, such as antibodies, proteins, and some peptides, for example. Exposure of an acridinium ester label to an alkaline solution cleaves the ester linkage to release an unstable compound that decomposes to trigger a flash of luminescent light emitted at a well-defined wavelength peak (hereinafter emission peak). Luminescence, as defined herein, is light emission from a substance when it returns from an excited state to its ground state.

However, existing luminescent detection systems may be insufficient in some respects in as far as they produce luminescence intensity that can be insufficient. Hence, improved systems and methods for luminescence detection, such as in immunoassay systems, are desired.

US 9,255,885 B2 discloses a luminescence detection system comprising capillary channels of a micro-sampler for transferring a body fluid onto the test field of a test element, which produces diffusely scattered light when irradiated with light from a light source. The scattered light is registered by an image detector such as a standard CCD/CMOS image sensor chip which typically exhibits a maximum efficiency in the range of 600 to 900 nm. A wavelength-converting material such as a fluorescent material is provided which e.g. converts photons having a wavelength of 360 nm into photons of 600 nm, whereby the wavelength conversion increases the detection efficiency due to a better sensitivity of the image sensor chip at longer wavelengths.

### SUMMARY

The present invention relates to luminescence detection systems as well as methods of luminescence detection as set out in the appended set of claims.

Numerous other aspects are provided in accordance with these and other aspects of the disclosure. Other features and aspects of the present disclosure will become more fully apparent from the following detailed description, the claims, and the accompanying drawings.

### BRIEF DESCRIPTION OF THE DRAWINGS

FIG. 1A is a schematic diagram of a chemiluminescence detection system according to embodiments of the present disclosure.
FIG. 1B is an enlarged, cross-sectioned, schematic diagram of a portion of the chemiluminescence detection system of FIG. 1A according to embodiments of the present disclosure.
FIG. 1C is an enlarged, cross-sectioned, schematic diagram of a portion of an alternative chemiluminescence detection system including a slide-in substrate including a conversion coating thereon according to embodiments of the present disclosure.
FIG. 2A is a spectral plot of luminescent emissions (e.g., red light emissions) from a processed specimen contained in a holder and including a red dye label that is tagged to the component according to embodiments of the present disclosure.
FIG. 2B is a spectral plot of quantum efficiency (%) versus wavelength (nm) of a photomultiplier tube (PMT) in a luminescent detection system according to embodiments of the present disclosure.
FIG. 2C is a spectral plot of luminescent emissions that have been down-converted to a second wavelength range by operation of a conversion coating adjacent to the light entrance window according to embodiments of the present disclosure.
FIG. 3 is a spectral plot of quantum efficiency (%) and down-converted luminescent emissions both versus wavelength (nm) illustrating the incident peak of the converted luminescence falling within the maximum detection efficiency wavelength range according to embodiments of the present disclosure.
FIG. 4 is a flowchart depicting an example method of luminescence detection according to embodiments of the present disclosure.

### DETAILED DESCRIPTION

In view of the above expressed issues and concerns, systems and methods that have relatively improved sensitivity are provided. Chemiluminescence technology in immunoassays relies on chemiluminescent tags at chemiluminescent labels that have high quantum yields at tag- and conjugate-specific emission wavelengths. Current photo multiplier tube (PMT) detectors used to read the chemiluminescence emissions have a maximum sensitivity at a specific wavelength (a maximum quantum efficiency %). Unfortunately, as the inventors have recognized, this maximum quantum efficiency may not be at the same wavelength as the highest quantum yield of chemiluminescence emissions. Thus, the sensitivity of detection of existing chemiluminescence detection systems may be lacking.

In particular, prior systems have used, for example, a blue dye label (e.g., with a nominal emission wavelength at from about 398 nm to 420 nm) as it best matches with the wavelength of maximum quantum efficiency (%) of the detector, which is at a wavelength of about 375 nm. However, the inventors have further recognized that light intensity or energy flux is proportional to photon flux, i.e., the number of photons per unit time per unit surface and not to individual photon energy. Thus, the number of photons at lower frequency (higher wavelength) will be more than that at higher frequency (lower wavelength). Thus, photon flux from using a blue dye label can be improved, for example by using a dye label having a higher luminescent emission wavelength range, such as a red dye label. However, when a red dye label is used, the quantum efficiency of the conventional sensor is reduced, as the luminescent emission wavelength moves away from the maximum quantum efficiency. In fact, the quantum sensitivity can be reduced more than a hundred fold by switching to a red dye label.

Thus, in order to improve sensitivity of detection, and in accordance with a first aspect of the disclosure, the chemiluminescent emissions should be matched with the detector's maximum sensitivity wavelength. However, this is a challenge with existing systems as blue label detectors are as stated above have relatively low photon flux. Thus, in existing luminescent detection systems there are losses of sensitivity due to the mismatch between the incident luminescent spectrum and the spectral absorption properties of the detector. Intensities for various assays are read out using the single detector, irrespective of their maximum quantum yield, thus losing intensity and impacting sensitivity for at least some wavelengths. The resulting lower sensitivity is simply tolerated in existing systems.

Thus, in accordance with one aspect, a luminescence detection system is provided that can sensitively measure luminescent assay emissions. In some embodiments, the luminescence detection system can be implemented on existing analyzers as a hardware retrofit upgrade. For example, the luminescence detection system can replace the existing luminescence detection system utilized in the analyzer. In accordance with one or more features of the disclosure, the improved luminescence detector system allows photons to pass through that are already in the maximum detection efficiency wavelength range (maximum detection window). However, photons that lie outside the maximum detection efficiency wavelength range (max detection window) are converted to fall into the maximum detection efficiency wavelength range (max detection window). Thus, minimum intensity loss can be incurred when using the conversion.

Thus in accordance with aspects of the present disclosure, higher total read-out of photoluminescent immunoassays can be achieved, resulting in higher sensitivity. Furthermore, aspects of the present disclosure allow the option to use higher quantum yield labels (e.g., red dye labels) that are currently out of range for conventional instrumentation. Additionally, aspects of the present disclosure allow for the conversion of "waste photons" into detected photons by use of a photoconversion layer (conversion layer) added to, or used in conjunction with, the photodetector. Thus, a greater range of immunoassays, with potentially a higher sensitivity, can be used with the luminescence detection systems of the present disclosure.

Thus, according to embodiments of the disclosure, luminescence detection systems are provided that can be implemented within immunoassay instruments to provide improved intensity of luminescent emission as well as improved luminescent emission detection sensitivity. Further, luminescence detection methods adapted to provide improved detection of luminescence emitted from a chemiluminescent reaction are provided.

Thus, it should be recognized that the methods and systems described herein cannot only improve the intensity of luminescent light emission, but at the same time can improve overall detection sensitivity. Thus, overall signal strength of the luminescence detection system can be dramatically improved.

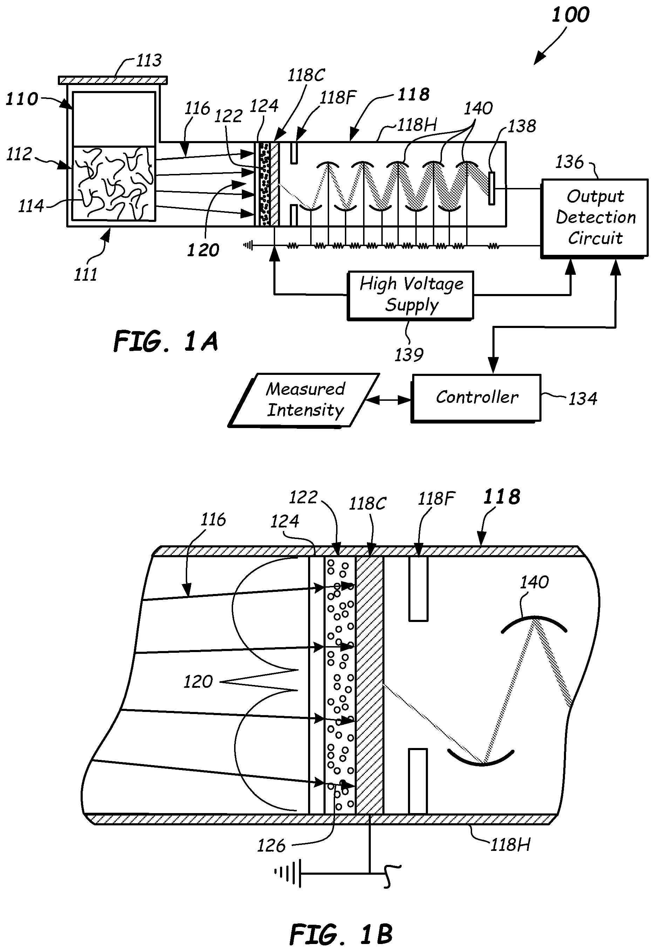

Further details and examples of apparatus, systems, and methods of the disclosure are provided with reference to FIGs. 1A-4 herein.

FIGs. 1A-1B depicts an example embodiment of a luminescence detection system 100 that is configured and operable to carry out the method to measure intensity of luminescent light (e.g., luminescent light emissions) emanating from a chemiluminescent reaction of labeled components 114 provided in a solution 112 of a test sample contained in a sample holder 110 located at a sample location 111 within the luminescence detection system 100. The solution 112 of test sample comprises a solution containing the labeled component 114 plus a solution that has been added to cause the chemiluminescent reaction with the labeled component. Thus, the sample holder 110 is configured to hold, in solution 112, a test sample containing the labeled components 114, wherein the component has been obtained from a processed biological specimen. The biological specimen can be any bio-fluid, such as blood serum, blood plasma, urine, cerebrospinal fluid, or the like. The sample holder 110 can be a cuvette or other vessel that is optically transparent or translucent, such as a plastic or glass. The walls of the sample holder 110 may be planar or can be curved or combinations thereof. The sample holder 110 may be provided at the sample location 111, by entry through a door or lid 113 or other suitable introduction methods.

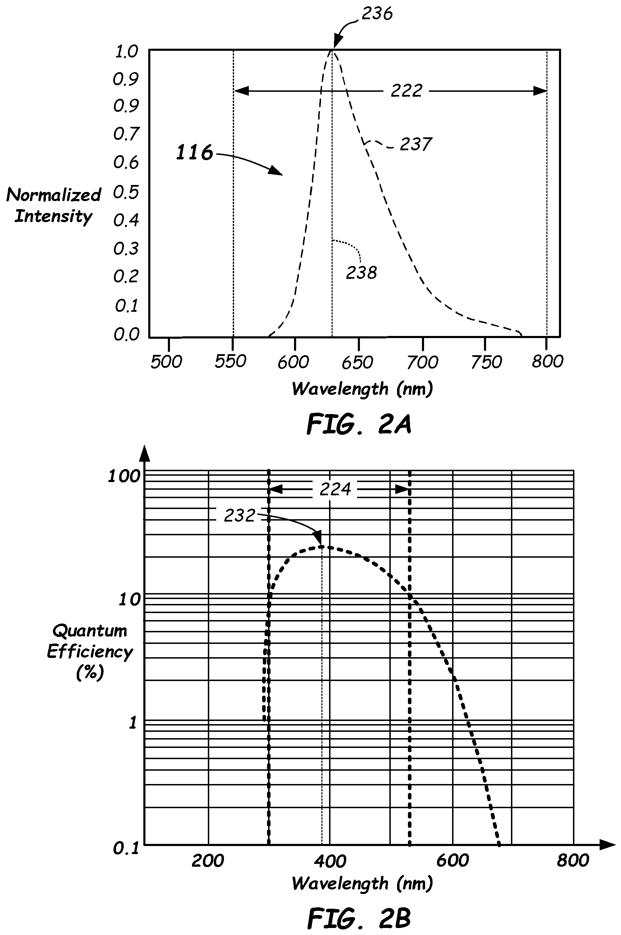

The dye label can be any suitable dye label that undergoes a chemiluminescent reaction and thus emits luminescent emissions 116 over a first wavelength range. For example, the dye label can emit luminescent emissions 116 over a wavelength range 222 from 550 nm to 800 nm, as is shown in FIG. 2A, for example. Further, the luminescent emissions 116 can have a spectral response having a luminescent peak 236 and a spectral distribution of luminescent intensity 237 about the luminescent peak 236, as shown. This particular dye label has a luminescent peak 236 at between about 605 nm and about 650 nm. The spectral distribution of luminescent intensity 237 can be a non-normal distribution as shown, where greater that 50% of the normalized intensity lies above the luminescent peak 236.

Thus, the labeled component 114 undergoes a chemiluminescent reaction upon addition of a suitable agent and emits luminescent emissions 116 over the first wavelength range 222. The labeled component can be any labeled analyte of interest, such as a labeled antibody, a labeled autoantibody, a labeled antigen, a labeled protein, a labeled DNA probe, a labeled marker, and the like. Nucleic acid probes and haptens may also be labeled. Labeling can be accomplished indirectly by binding conjugates or directly by direct enzyme conjugation. For example, acridinium esters are direct chemiluminescent labels for antibodies and DNA probes. Acridinium esters used as direct labels can be attached to the probe through a hybridization reaction. Acridinium esters can be reacted with alkaline peroxide (e.g., hydrogen peroxide) under basic conditions to yield an excited state, which emits light at a defined wavelength. Derivatives such as acridinium sulfonamide ester labels may also be used. Other types of luminophore and enzymatic labels may be used. The present method can be used for identifying and detecting concentrations of various components (e.g., antibodies, autoantibodies for diagnosis of autoimmune diseases, serum concentrations of hormones, polypeptides, drugs, vitamins, tumor markers, infectious disease markers, inflammation markers, myocardial damage markers, and the like) as a biochemical technique used in immunology.

Again referring to FIGs. 1A and 1B, luminescence detection system 100 further includes a photodetector 118, such as a photomultiplier tube (PMT), having a light entrance window 120 that is configured to receive at least a portion of the luminescent emissions 116 (photons) emitted from the chemiluminescent reaction. Light entrance window 120 may have a circular shape in plan view, and a receiving area that is sufficiently large, for example. Other shapes are possible. As shown in FIG. 2B, the photodetector 118 has a maximum detection efficiency wavelength range 224. The maximum detection efficiency wavelength range 224 is the range over which the quantum efficiency is 10% or greater, as shown for example. However, in some portions of the maximum detection efficiency wavelength range 224, the quantum efficiency can be 15% or more, 20% or more, or even 22% or more.

As can be seen from FIG. 2B, the maximum detection efficiency wavelength range 224 of the photodetector 118 is fairly efficient at receiving blue luminescent light emissions, which have a wavelength range from about 398 nm and 420 nm. However, blue dye labels emit comparatively low photon emission intensities, and thus cannot provide high detection levels. Red dye labels, however, have much higher photon emission intensities, but as can be seen from FIG. 2A, red light which has emissions at from about 550 nm to 800 nm would have quantum efficiencies that are too low, e.g., below about 1%. Thus, using a red dye label with a blue-dominant detector would result in very low detection sensitivity.

However, according to various embodiments herein, as shown in FIG. 1A and enlarged FIG. 1B, a conversion coating 122 is provided at a location adjacent to the light entrance window 120. For example, in a first embodiment, the conversion coating 122 can be applied directly to a substrate comprising a photocathode 118C in light entrance window 120 of the photodetector 118.

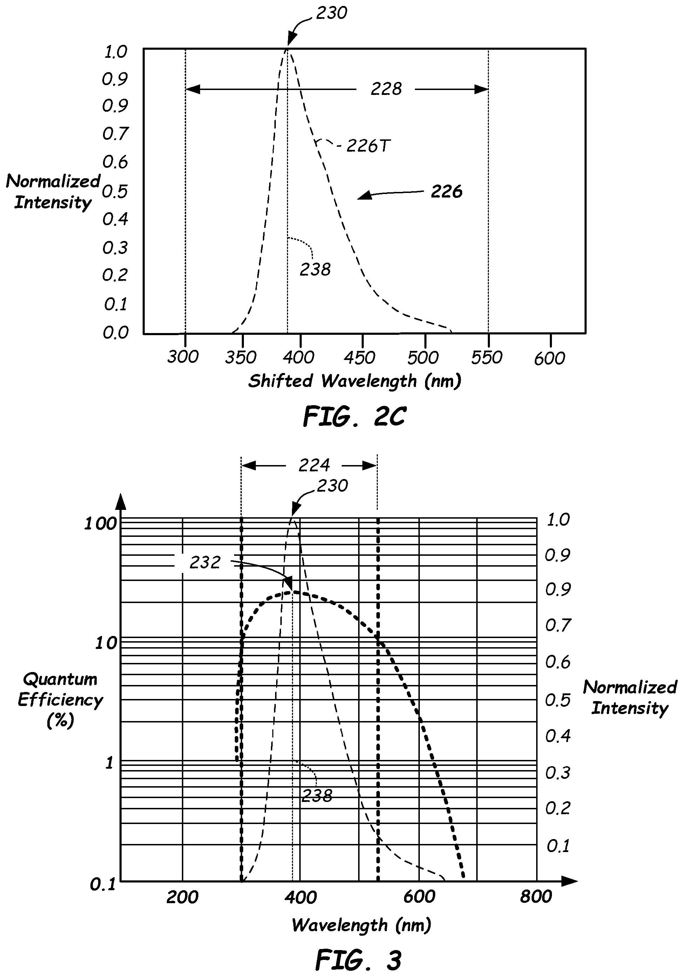

The conversion coating 122 functions to convert at least some of the luminescent emissions 116 to incident emissions 126 that have been shifted in wavelength to fall within a second wavelength range 228 as shown in FIG. 2C. The second wavelength range 228 can be from 300 nm to 550 nm or even from 325 nm to 525 nm in some embodiments. As shown, the incident emissions 126 (photons) that contact the photocathode 118C of the photodetector 118 can have an incident peak 230 (FIG. 2C). Photocathode 118C converts incident photons into electrons.

The conversion coating 122 is designed so that the incident peak 230 falls within the maximum detection efficiency wavelength range 224 of the photodetector 118 as best shown in FIG. 3. Thus, effectively, red-dominant light has been converted or shifted to blue-dominant light in this embodiment. Thus, a double advantage of higher photon emission generated via the use of red dominant light is achieved along with improved sensitivity by downshifting so that the incident peak 230 falls within the maximum detection efficiency wavelength range 224. Maximum detection efficiency wavelength range 224 can be from 300 nm to 530 nm, for example. In some embodiments, it is desired that the incident peak 230 substantially coincides with the location of the quantum efficiency peak 232 within the maximum detection efficiency wavelength range 224. By "substantially coincide," it is meant that the two peaks 230, 232 differ in location by no more than 50 nm, so that maximum or near maximum detection sensitivity is achieved. The detection using the photodetector 118 can take place at one or more suitable time increments after introduction of the agent to cause the chemiluminescent reaction.

Again referring to FIG. 1A, the luminescence detection system 100 further includes, and may be controlled by, a suitable controller 134. Controller 134 can include a suitable processor and memory for storing signals obtained from an output detection circuit 136. The output of the output detection circuit 136 can be provided from an anode 138 at the end of a series of dynode stages 140. Electrons are ejected from the surface of the photocathode 118C as a consequence of the photoelectric effect. The absorbed energy causes electron emission. These electrons are directed by the focusing electrode 118F toward the electron multiplier comprising the dynode stages 140, where electrons are multiplied by the process of secondary emission. Such an arrangement of dynode stages 140 is able to amplify the small current emitted by the photocathode 118C, typically by a factor of one million or more. Output detection circuit 136 can then measure the resulting current at the cathode 138, which provides an estimate of the amount of spectral luminescent emissions. Any suitable conventional output detection circuit and high voltage supply 139 may be used.

The photodetector 118 comprising a photomultiplier tube (PMT) can be constructed with an evacuated glass housing surrounding the series of dynode stages 140. The conversion coating 122 may be designed so that the luminescent peak 236 is shifted via the luminescent emissions 116 passing through the conversion coating 122 to become incident emissions 126 colliding with the photocathode 118C. In particular, the wavelength shift may be sufficient to bring the shifted incident peak 230 within the maximum detection efficiency wavelength range 224. In some embodiments, the shift may be 100 nm or more, 120 nm or more, or even 150 nm or more, for example.

The conversion coating 122 may be provided in the form of a transparent carrying matrix with suspended dye molecules or quantum dots, for example. The coating of the conversion coating 122 may be achieved by direct coating of the light entrance window 120 with the conversion coating 122. Optionally, in an alternative embodiment, a transparent substrate 123 (e.g., window pane-like element) can include the conversion coating 122 applied thereon and can be installed in front of the photocathode 118C, such as by receiving the transparent substrate 123 in a slot 127. Each of these embodiments may be retrofittable to existing PMT detectors.

The conversion coating 122 may be a thin 10 nm to 100 nm transparent layer provided with included conversion elements (suspended dye molecules or quantum dots). A reflective member 124, such as reflective coating (e.g., a narrow-band reflecting coating) may be applied overtop of the conversion coating 122. The reflective member 124 may have a narrow band surrounding and which may be centered on the quantum efficiency peak 232 (FIG. 2B) of the photocathode 118c. In some embodiments, the reflective member 124 can comprise a long pass dichroic mirror that lets luminescent emissions 116 over the first wavelength range 222 pass through and rejects at least some light outside of the first wavelength range 222. This may aid in avoiding absorption in other parts of the spectrum. In further embodiments, the reflective member 124 comprises a universal detector that allows all light emissions through, but reflects and back-reflects incident emissions 126 of a second wavelength range 228 towards the photodetector 118.

Whatever dye is chosen, and for whatever photodetector is chosen for the luminescent detection system, the luminescent emissions can be shifted in wavelength to substantially coincide with the maximum quantum efficiency range 224 of the photodetector 118. Any suitable up conversion coating may be used.

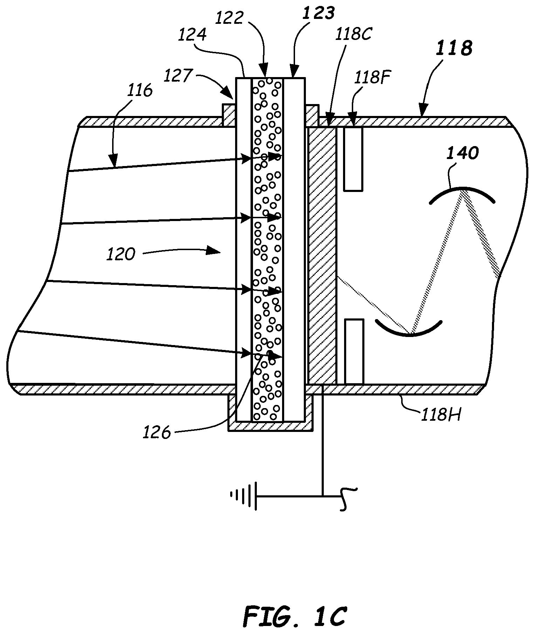

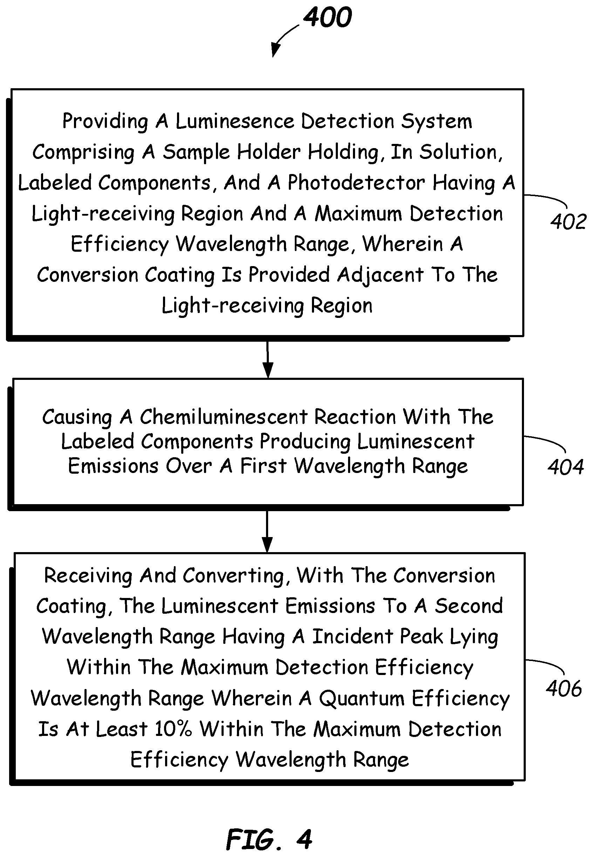

FIG. 4 illustrates a flowchart depicting a method 400 of luminescence detection for use in, for example, immunoassay testing. The method 400 comprises, in block 402, providing a luminesence detection system (e.g., luminesence detection system 100) comprising a sample holder (e.g., sample holder 110) holding, in solution (e.g., solution 112), labeled components (e.g., labeled components 114) from a biological sample, and a photodetector (e.g., photodetector 118) having a light-receiving region (e.g., light entrance window 120) and a maximum detection efficiency wavelength range (e.g., maximum detection efficiency wavelength range 224), wherein a conversion coating (e.g., conversion coating 122) is applied adjacent to the light-receiving region (e.g., light entrance window 120). The conversion coating 122 can be applied directly on the face of the cathode 118C. Optionally, the conversion coating 122 can be applied as a coating on a transparent substrate 123 provided in front of the cathode 118C, such as a slide-in transparent glass panel coated with the conversion coating 122 shown in FIG. 1C.

The method 400 further comprises, in block 404, causing a chemiluminescent reaction with the labeled components producing luminescent emissions (e.g., luminescent emissions 116) over a first wavelength range, wherein the first first wavelength range may be from 550 nm to 800 nm, for example. Other suitable ranges may be implemented provided that a dye label undergoes luminescent emissions in that range.

The method 400 further includes, in block 406, receiving and converting, with the conversion coating (e.g., conversion coating 122), the luminescent emissions (e.g., luminescent emissions 116) to a second wavelength range (second wavelength range 228) having a incident peak (e.g., incident peak 230) lying within the maximum detection efficiency wavelength range (e.g., maximum detection efficiency wavelength range 224) wherein a quantum efficiency (%) is at least 10% within the maximum detection efficiency wavelength range. In some embodiments, the maximum detection efficiency wavelength range is from 300 nm to 530 nm.

Although embodiments are described herein with reference to specific examples, the scope of the disclosure is not intended to be limited to the details and specific examples described herein. Rather, various modifications may be made to the embodiments and details within the scope of the claims.

## Claims

1. A luminescence detection system, comprising:
a sample holder (110) configured to hold, in solution, a test sample (112) including labeled components (114), wherein the labeled components in the test sample undergo a chemiluminescent reaction and emit luminescent emissions over a first wavelength range, which range is from 550 nm to 800 nm;
a photodetector (118) having a light entrance window (120) configured to receive light emissions, the photodetector having a maximum detection efficiency wavelength range, which range is from 300 nm to 530 nm; and
a conversion member (122) provided adjacent to the light entrance window of the photodetector, wherein the conversion member operates to cause a conversion of the luminescent emissions over the first wavelength range to incident emissions of a second wavelength range, which range is from 300 nm to 550 nm, wherein an incident peak of the incident emissions falls within the maximum detection efficiency wavelength range, wherein the maximum detection efficiency wavelength range is a range of wavelengths where a quantum efficiency of the photodetector is 10% or more.

2. The luminescence detection system of claim 1, wherein the conversion member comprises a conversion coating applied directly to the light entrance window, or wherein the conversion member comprises a conversion coating that is applied to a transparent substrate located in front of the light entrance window.

3. The luminescence detection system of claim 1, wherein the conversion is an up-conversion.

4. The luminescence detection system of claim 1, wherein the quantum efficiency is 15% or more, preferably 20% or more, more preferably 22% or more.

5. The luminescence detection system of claim 1, wherein the first wavelength range has a luminescent peak at from 605 nm and 650 nm.

6. The luminescence detection system of claim 1, wherein the second wavelength range is from 325 nm to 525 nm.

7. The luminescence detection system of claim 1, wherein the photodetector comprises a photomultiplier tube.

8. The luminescence detection system of claim 1, comprising a reflective member that is provided in front of the conversion member.

9. The luminescence detection system of claim 9, wherein the reflective member comprises a long pass dichroic mirror that lets the luminescent emissions over a first wavelength range pass through and rejects at least some light outside of the first wavelength range, or wherein the reflective member comprises a universal detector that allows all of the luminescent emissions through, but back-reflects incident emissions of a second wavelength range towards the photodetector.

10. A method of luminescence detection, comprising:
providing a luminescence detection system comprising a sample holder (110)
holding, in solution (112), labelled components (114), and a photodetector (118) having a light receiving region (120) and a maximum detection efficiency wavelength range which range is from 300 nm to 530 nm, wherein a conversion coating (122) is applied adjacent to the light-receiving region;
causing a chemiluminescent reaction with the labeled components producing luminescent emissions over a first wavelength range which range is from 550 nm to 800 nm; and
receiving and converting, with the conversion coating, the luminescent emissions to a second wavelength range which range is from 300 nm to 550 nm, and having an incident peak lying within the maximum detection efficiency wavelength range wherein a quantum efficiency is at least 10% within the maximum detection efficiency wavelength range.

## Patentansprüche

1. Lumineszenzdetektionssystem, umfassend:
einen Probenhalter (110), der dazu ausgelegt ist, eine Testprobe (112), die markierte Komponenten (114) einschließt, in Lösung zu halten,
wobei die markierten Komponenten in der Testprobe eine Chemilumineszenzreaktion eingehen und Lumineszenzemissionen über einen ersten Wellenlängenbereich emittieren, der von 550 nm bis 800 nm beträgt;
einen Photodetektor (118) mit einem Lichteintrittsfenster (120), das dazu ausgelegt ist, Lichtemissionen zu empfangen, wobei der Photodetektor einen Wellenlängenbereich mit maximaler Detektionseffizienz aufweist, der von 300 nm bis 530 nm reicht; und
ein Konvertierungselement (122), das angrenzend an das Lichteintrittsfenster des Photodetektors bereitgestellt ist, wobei das Konvertierungselement arbeitet, um eine Konvertierung der lumineszierenden Emissionen über den ersten Wellenlängenbereich in einfallende Emissionen eines zweiten Wellenlängenbereichs zu bewirken, dessen Bereich von 300 nm bis 550 nm beträgt, wobei ein einfallender Peak der einfallenden Emissionen in den Wellenlängenbereich mit maximaler Detektionseffizienz fällt, wobei der Wellenlängenbereich mit maximaler Detektionseffizienz ein Bereich von Wellenlängen ist, wo eine Quanteneffizienz des Photodetektors 10 % oder mehr beträgt.

2. Lumineszenzdetektionssystem nach Anspruch 1, wobei das Konvertierungselement eine Konvertierungsbeschichtung umfasst, die direkt auf das Lichteintrittsfenster aufgebracht ist, oder wobei das Konvertierungselement eine Konvertierungsbeschichtung umfasst, die auf ein transparentes Substrat aufgebracht ist, das sich vor dem Lichteintrittsfenster befindet.

3. Lumineszenzdetektionssystem nach Anspruch 1, wobei die Konvertierung eine Aufwärtskonvertierung ist.

4. Lumineszenzdetektionssystem nach Anspruch 1, wobei die Quanteneffizienz 15 % oder mehr, vorzugsweise 20 % oder mehr, bevorzugter 22 % oder mehr beträgt.

5. Lumineszenzdetektionssystem nach Anspruch 1, wobei der erste Wellenlängenbereich einen Lumineszenzpeak bei von 605 nm bis 650 nm aufweist.

6. Lumineszenzdetektionssystem nach Anspruch 1, wobei der zweite Wellenlängenbereich von 325 nm bis 525 nm beträgt.

7. Lumineszenzdetektionssystem nach Anspruch 1, wobei der Photodetektor eine Photovervielfacherröhre umfasst.

8. Lumineszenzdetektionssystem nach Anspruch 1, umfassend ein reflektierendes Element, das vor dem Konvertierungselement bereitgestellt ist.

9. Lumineszenzdetektionssystem nach Anspruch 9, wobei das reflektierende Element einen dichroitischen Langpass-Spiegel umfasst, der die Lumineszenzemissionen über einen ersten Wellenlängenbereich hindurch lässt und mindestens etwas Licht außerhalb des ersten Wellenlängenbereichs abweist, oder wobei das reflektierende Element einen universellen Detektor umfasst, der alle Lumineszenzemissionen durchlässt, aber einfallende Emissionen eines zweiten Wellenlängenbereichs in Richtung des Photodetektors zurückreflektiert.

10. Verfahren zur Lumineszenzdetektion, umfassend:
Bereitstellen eines Lumineszenzdetektionssystems, umfassend einen Probenhalter (110), der markierte Komponenten (114) in Lösung (112) hält, und einen Photodetektor (118) mit einer Lichtempfangsregion (120) und einem Wellenlängenbereich mit maximaler Detektionseffizienz, der im Bereich von 300 nm bis 530 nm liegt, wobei eine Konvertierungsbeschichtung (122) angrenzend an die Lichtempfangsregion aufgebracht wird;
Bewirken einer Chemilumineszenzreaktion mit den markierten Komponenten, die Lumineszenzemissionen über einen ersten Wellenlängenbereich produzieren, der von 550 nm bis 800 nm reicht; und
Empfangen und Konvertieren der Lumineszenzemissionen in einen zweiten Wellenlängenbereich, der von 300 nm bis 550 nm reicht, mit der Konvertierungsbeschichtung, und wobei ein einfallender Peak innerhalb des Wellenlängenbereichs mit maximaler Detektionseffizienz liegt, wobei eine Quanteneffizienz innerhalb des Wellenlängenbereichs mit maximaler Detektionseffizienz mindestens 10 % beträgt.

## Revendications

1. Système de détection de luminescence comprenant :
un porte-échantillon (110) configuré pour contenir, en solution, un échantillon d'essai (112) comprenant des composants marqués (114), dans lequel les composants marqués dans l'échantillon d'essai subissent une réaction chimioluminescente et émettent des émissions luminescentes sur une première plage de longueurs d'onde, comprise entre 550 nm et 800 nm ;
un photodétecteur (118) ayant une fenêtre d'entrée de lumière (120) configurée pour recevoir des émissions lumineuses, le photodétecteur ayant une plage de longueurs d'onde d'efficacité de détection maximale, cette plage est comprise entre 300 nm et 530 nm ; et
un élément de conversion (122) pourvu adjacent à la fenêtre d'entrée de lumière du photodétecteur, dans lequel l'élément de conversion fonctionne pour provoquer une conversion des émissions luminescentes sur la première plage de longueurs d'onde en émissions incidentes d'une deuxième plage de longueurs d'onde, comprise entre 300 nm et 550 nm, dans lequel un pic des émissions incidentes se situe dans la plage de longueurs d'onde d'efficacité de détection maximale, dans lequel la plage de longueurs d'onde d'efficacité de détection maximale est une plage de longueurs d'onde dans lequel l'efficacité quantique du photodétecteur est supérieure ou égale à 10 %.

2. Système de détection de luminescence de la revendication 1, dans lequel l'élément de conversion comprend un revêtement de conversion appliqué directement sur la fenêtre d'entrée de lumière, ou dans lequel l'élément de conversion comprend un revêtement de conversion appliqué sur un substrat transparent situé devant la fenêtre d'entrée de lumière.

3. Système de détection de luminescence de la revendication 1, dans lequel la conversion est une conversion vers le haut.

4. Système de détection de luminescence de la revendication 1, dans lequel l'efficacité quantique est de 15 % ou plus, de préférence de 20 % ou plus, de préférence encore de 22 % ou plus.

5. Système de détection de luminescence de la revendication 1, dans lequel la première plage de longueurs d'onde présente un pic de luminescence entre 605 nm et 650 nm.

6. Système de détection de luminescence de la revendication 1, dans lequel la deuxième longueur d'onde est comprise entre 325 nm et 525 nm.

7. Système de détection de luminescence de la revendication 1, dans lequel le photodétecteur comprend un tube photomultiplicateur.

8. Système de détection de luminescence de la revendication 1 comprend un élément réfléchissant qui est pourvu devant l'élément de conversion.

9. Système de détection de luminescence de la revendication 9, dans lequel l'élément réfléchissant comprend un miroir dichroïque à grand passage qui laisse passer les émissions luminescentes sur une première plage de longueurs d'onde et rejette au moins une partie de la lumière en dehors de la première plage de longueurs d'onde, ou dans lequel l'élément réfléchissant comprend un détecteur universel qui laisse passer toutes les émissions luminescentes, mais renvoie les émissions incidentes d'une deuxième plage de longueurs d'onde vers le photodétecteur,

10. Procédé de détection de luminescence, comprenant les étapes consistant à :
fournir un système de détection de luminescence comprenant un porte-échantillon (110) contenant, en solution (112), des composants marqués (114), et un photodétecteur (118) ayant une région de réception de lumière (120) et une plage de longueurs d'onde d'efficacité de détection maximale comprise entre 300 nm et 530 nm, dans lequel un revêtement de conversion (122) est appliqué à côté de la région de réception de la lumière ;
provoquer une réaction chimioluminescente avec les composants marqués produisant des émissions luminescentes sur une première plage de longueurs d'onde allant de 550 nm à 800 nm ; et
recevoir et convertir, avec le revêtement de conversion, les émissions luminescentes sur une deuxième plage de longueurs d'onde allant de 300 nm à 550 nm, et ayant un pic incident situé dans la plage de longueurs d'onde d'efficacité de détection maximale, dans lequel une efficacité quantique est d'au moins 10 % dans la plage de longueurs d'onde d'efficacité de détection maximale.
